# EUROPEAN PATENT APPLICATION

(11) **EP 3 838 133 A1**
(43) Date of publication of application: **23.06.2021**
(21) Application number: 19306727.9
(22) Date of filing: 20.12.2019
(51) Int. Cl.: A61B 5/0408, A61B 5/0456, A61B 5/0468, A61B 5/0472, G16H 50/20

(54) **DEVICE AND PROCESS FOR ECG MEASUREMENTS**

(71) Applicant: Kaptalia Monitoring, 56000 Vannes (FR)
(72) Inventor: OBEID, Dany, 56000 Vannes (FR); DUMAND, Jean-Bernard, 56000 Vannes (FR)
(74) Representative: Plasseraud IP

(57) **Abstract**

Process for measuring heart beats fiducial points and classifying heart beats, comprising:
- sampling (100a) a raw ECG signal (100) acquired with an ECG sensor (10) on a patient,
- providing (130a) a first filtered signal (102) through removing the baseline wander from said sampled raw ECG signal with a baseline removal filter module (130),
- providing (110a) a second filtered signal (101) through a bandpass filter module (110) comprising a bandpass filter and derivation, squaring and moving window integration submodules providing a filtered signal for a beat detection module (120)
- detecting (120a) left and right limits data (125) for the beats in said second filtered signal within said beat detection module,
- in a Fiducial point extraction module (150), receiving the first filtered signal (102) from the baseline filter removal module (130) and receiving said left and right limits data (125) from said beat detection module (120), sampling and storing (150a) ECG curve data of beats from said first filtered signal synchronized by said left and right limits data and extracting (150b) fiducial points of said beats from said ECG curve data (150), said fiducial points comprising at least the QRS points values of the beats, and classifying (160a) each of said beats in classes based on a correlation of its ECG curve data with respect to average ECG curves data of classes of previously averaged (600) classified beats.

## Description

### Field of the invention

The present application pertains to the field of signal processing of ECG curves and provides a device and process for ECG measurements having means for detecting signals corresponding to Premature Ventricular Complexes (PVC) which may indicate occurrences of premature ventricular contractions.

### Background art

Detection techniques for PVCs in ECG curves include artificial neural network, linear classification methods, real-time detection systems using wavelet transform, energy analysis by moving window. Examples of methods can be found in:
Omer T. Inan, Laurent Giovangrandi and Gregory T. A. Kovacs, "Robust Neural-Network-Based Classification of Premature Ventricular Contractions Using Wavelet Transform and Timing Interval Features," in IEEE Transactions on Biomedical Engineering, vol. 53, no. 12, pp. 2507 2515, Dec. 2006. doi: 10.1109/TBME.2006.880879;
John S. Lim, "Finding Features for Real-Time Premature Ventricular Contraction Detection Using a Fuzzy Neural Network System" in IEEE Transactions on Neural Networks, vol. 20, no. 3, pp. 522-527, March 2009. doi: 10.1109/TNN.2008.2012031 ;
Saurabh Pal, Madhuchhanda Mitra: "Detection of Premature Ventricular Contraction Beats Using ANN" International Journal of Recent Trends in Engineering and Technology, vol. 3, no. 4, May 2010.
Robert Chen-Hao Chang, Chih-Hung Lin, Ming-Fan Wei, Kuang-Hao Lin, and Shiue-Ru Chen, "High-Precision Real-Time Premature Ventricular Contraction (PVC) Detection System Based on Wavelet Transform", Journal of Signal Processing Systems DOI 10.1007/s11265-013-0823-6, December 2014;
Ernano Arrais Junior, Ricardo Alexsandro de Medeiros Valentim, Gláucio Bezerra Brandão, "Real-time premature ventricular contractions detection based on Redundant Discrete Wavelet Transform", Research on Biomedical Engineering, 2018; volume 34, number 3 DOI: 10.1590/2446-4740.01618;
J. Pan and W. J. Tompkins, "A Real-Time QRS Detection Algorithm," in IEEE Transactions on Biomedical Engineering, vol. BME-32, no. 3, pp. 230-236, March 1985. doi: 10.1109/TBME.1985.325532;
GB Moody, RG Mark. "The impact of the MIT-BIH Arrhythmia Database." IEEE Engineering in Medicine and Biology 20(3):45-50 (May-June 2001). (PMID: 11446209).

### Technical problem

The use of wavelet transform has permitted to improve the PVC detection sensitivity and precision drastically in the past years on the MIT data sample files. However, these known methods need large calculation capabilities and powerful computer systems. Efficient ECG monitoring systems usually have a power consumption of at least 50W which is far too high for carry-on devices. In addition, PVC detection systems need at least three electrodes for filtering the signal and the processing time is about 60 seconds to analyze a signal of 30 min which is not appropriate for emergency situations.

### Summary of the Invention

In view of such issue, the present disclosure aims to provide a low-power wireless standalone multi-parameters monitoring system that can be easily used in emergency medical services and patient monitoring in motion. This system includes an ECG analyser that automatically detects abnormal ECG curves and triggers their related alarms signals with analysis methods which require less calculation power than prior art systems thus allowing the use of a low power tablet device and allowing real time detection.

In this regard, in a first aspect, the present disclosure provides a novel measurement process based on a specific real time processing algorithm which on the one end permits using a simple sensor with two electrodes in lieu of more complex three electrodes or more sensors and on the other hand drastically reduces the power consumption of the microprocessor of the monitoring system allowing using a tablet PC device connected to a wireless bipolar electrode device as the monitoring system.

More precisely, the invention provides a process for measuring heart beats fiducial points and classifying heart beats which comprises sampling a raw ECG signal acquired with an ECG sensor on a patient,
- providing a first filtered signal through removing the baseline wander from said sampled raw ECG signal with a baseline removal filter module,
- providing a second filtered signal through a bandpass filter module comprising a bandpass filter and derivation, squaring and moving window integration submodules providing a filtered signal for a beat detection module,
- detecting left and right limits data for the beats in said second filtered signal within said beat detection module,
- in a Fiducial point extraction module, receiving the first filtered signal from the baseline filter removal module and receiving said left and right limits darta from said beat detection module, sampling and storing ECG curve data of beats from said first filtered signal synchronized by said left and right limits data, extracting fiducial points of said beats from said ECG curve data, said fiducial points comprising at least the QRS points values of the beats, and classifying each of said beats in classes based on a correlation of its ECG curve data with respect to average ECG curves data of classes of previously averaged classified beats.

This process is efficient in term of energy saving and accuracy of ECG curves analysis and provides an accurate abnormal beat detection and classification.

A second aspect concerns a process for detecting abnormal beats comprising comparing sampled ECG curve data of a Beat Zone of a current beat with a set of conditions with respect to average Beat Zone ECG curve data of a dominant class of beats to provide a set of flags for abnormal beats.

The set of conditions provides a quick and easy way to classify beats which needs limited calculation resources.

A third aspect concerns a device for implementing the process of the invention.

In this regard, the present disclosure uses a novel measurement system based on using a simple sensor with two electrodes instead of more complex three electrodes or more sensors. This sensor is connected wirelessly to a monitor where a specific real-time processing algorithm is implemented. This algorithm is capable of processing ECG signals in real-time with a low power consumption. This allows the use of a low-weight tablet PC device as a monitor.

More precisely, the device comprises a remote sensor and a computer device, said sensor being provided with two electrodes adapted to measure electrical heartbeat signals on the skin of a patient and provided with an analog to digital converter and radio communication circuits to transmit numerical representations of said heartbeat signals to said computer device configured to communicate with said sensor and to compute said numerical representation.

Further optional characteristics which may be combined or alternate are provided in the hereunder paragraphs.

In the process for measuring heart beats fiducial points and classifying heart beats:
- The sensor being a two electrodes ECG, said sensor may be positioned on the chest of the patient in a Manubrium-Sternal-Nipple orientation.

The two electrodes sensor permits to adapt the position of the sensor on the chest of the patient and permits to keep space for defibrillator pads. In addition, the radio communication with the computer device avoids cables encumbering the space.
- Said fiducial points extraction may comprise further extraction of at least one of the maximum and minimum amplitude values of a beat, J-point through J-wave and T-wave.
- Sampling and storing ECG curve data of beats may comprise detecting a R point of said beats, setting a window around the detected R point of said beats, said window providing a Beat Zone having two sub-zones for covering the QRS complex curve of said beats, the process comprising analysing the sampled and stored ECG curve data during said Beat Zone to extract said fiducial points within said Beat Zone.
- Said Beat Zone may be defined to have a width sufficient to include a QRS complex curve of 80ms to 120ms including the J-point of an ECG complex after the end of the QRS complex curve at usual heart rates such width being further defined to avoid to take into account two beats with a beat period at a theoretical maximum of 300 beats per minute.
- Said Beat Zone may be a time window having a length between 200ms and 400ms and preferably 250ms, having a first sub-zone of 100ms length preceding a calculated R-wave maximum point, and having a second sub-zone of 150ms following said calculated R-wave maximum point.
- Classifying said beats may comprise a first learning phase comprising storing sampled ECG curve data of Beat Zones and calculating fiducial points of a limited number of beats from such ECG curve data to provide initial beat classes.
- Said process may comprise providing a first dominant beat class in said learning phase.
- Classifying said beats may comprise for subsequent beats after the learning phase, correlating the ECG curve data of the Beat Zone of a new beat with the average values of ECG curve data of the Beat Zone of beats in previous classes to determine the most similar class for this beat and wherein:
   - In case of correlation of the Beat Zone of such new beat with existing classes is lower than a limit value, and the maximum number of classes is not exceeded, a new class is created and the new beat becomes a member of it as long as the maximum number of classes is not reached
   - In case of correlation of the Beat Zone of such new beat with existing classes is lower than a limit value and the maximum number of classes is reached and the new beat does not match any of the existing classes, the new beat remains without class and receives a flag;
   - in case of correlation of the Beat Zone of such new beat with an existing class is higher than a limit value, the new beat is added to the class and the average values of ECG curve data of the class is recalculated.
- Said bandpass filter is preferably a stable finite impulse response filter of the 32nd order type.
- Said bandpass filter is preferably designed to order to have a frequency bandwidth of 8Hz to 25Hz using 33 coefficients and a filter delay is of less than 16 samples for an ECG signal sampled at 250Hz.
- Said process may comprise determining a dominant class, said dominant class comprising the higher number of classified beats in a rolling time window.
- Said rolling time window is between 3 to 6 minutes and preferably 5min.
- The classes may be limited to 100 beats, first in first out.
- An empty class or a class having only one beat after receipt of ten new beats is removed from the classification.

The process for detecting abnormal beats comprising the process of measuring heart beats fiducial points and classifying heart beats according to the invention may comprise further comparing ECG curve data of the Beat Zone of a current beat with a set of conditions with respect to average Beat Zone ECG curve data of the dominant class and/or previous beats to provide a set of flags for abnormal beats.

Process for detecting abnormal beats may comprise setting a multiform PVC warning flag upon detection of more than one PVC class of beats in said rolling time window.

The process for detecting abnormal beats may comprise further calculating and storing the time t_{RR} between current beat calculated R point and previous beat calculated R point and comparing the current point t_{RR} to the average t_{RRm} of the three previously calculated and stored time between R points (t_{RRn-1}, t_{RRn-2}, t_{RRn-3})/3 for providing a premature beat flag.

The premature beat flag may be provided when a last t_{RR} is smaller than t_{RRM}X0.875.

The process for detecting abnormal beats may comprise a validation tree combining said premature beat flag and flags of said set of flags for abnormal beats to raise warning flags upon premature ventricular contraction detections.

The computer device may comprise further display means provided for displaying said numerical representation of beat curves, classes of beats and warning messages upon detection of occurrences of abnormal beat curves.

The ECG sensor may be a remote ECG sensor provided with two electrodes adapted to measure electrical heartbeat signals on the skin of a patient and provided with an analog to digital converter and radio communication circuits to connect through radio communication said sensor to said computer device and transmit numerical representations of said heartbeat signals to said computer device.

The invention concerns also a computer program comprising instructions for implementing the process the invention when such program is executed by a computer.

The invention also provides non transitory medium readable by a computer on which is recorded a program for implemented the process of the invention when such program is executed by a computer.

### Brief description of the drawings

A detailed description of exemplary embodiments of the invention will be discussed hereunder in reference to the attached drawings where:
Figure 1A is a schematic view of a computer device of the present disclosure;
Figure 1B is a schematic view of a sensor of the present disclosure;
Figure 2 is a representation of a standard beat shape with fiducial points;
Figure 3A is a representation of a raw ECG curve and a filtered ECG curve with a specific bandpass filter of the present disclosure;
Figure 3B is a representation of Beat Zones as defined in the present disclosure;
Figures 4A and 4B are schematic representations of signal processing modules of the present disclosure;
Figure 5 is a representation of a classification process of beats of the present disclosure;
Figure 6 is a representation of a class update process of the present disclosure;
Figures 7A, 7B, 7C are parts of an abnormal beat detection process of the present disclosure;
Figure 8 is a schematic representation of the positioning of a sensor of the present disclosure on the chest of a patient;
Figures 9A and 9B are exemplary ECG curves.
Figures 10A to 10D are examples of curves at various steps of the treatment process;
Figures 11 and 12 are flowcharts of operations in the frame of the present disclosure;
Figure 13 is a simplified flowchart of block operations of the present disclosure

### Detailed description of embodiments of the invention

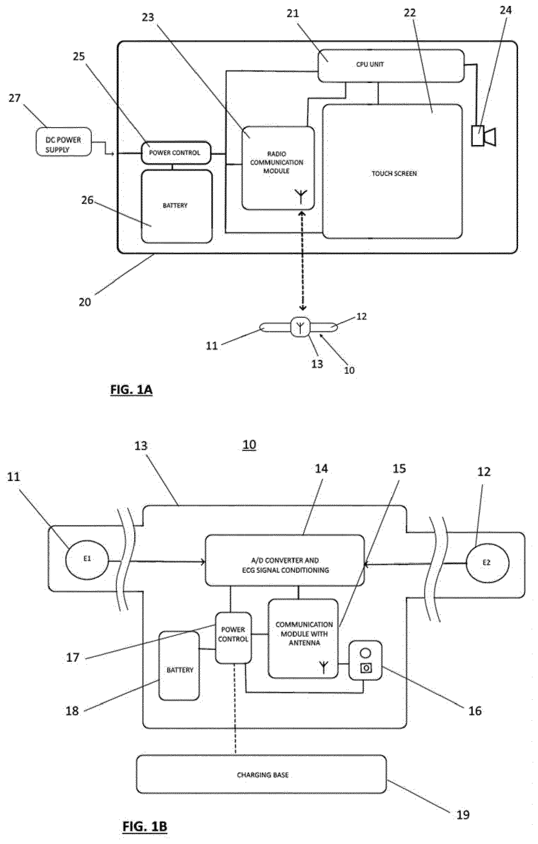

The device of the present disclosure comprises a hand-held device 20 and a bipolar sensor 10 connected through a wireless link as depicted in figure 1A.

The hand-held device 20 is in the form of a tablet PC comprising a low power CPU unit 21 with microprocessor, RAM, ROM and rewritable permanent memory such as SSD or other for handling measurement programs, a touch screen 22, a speaker 24, a radio communication module such as a Bluetooth module adapted to Bluetooth Low Energy communication with communication protocols adapted to communicate with the sensor 10, and a power controller to handle charging of the tablet battery.

The sensor 10 is more precisely described in figure 1B and comprises two electrodes 11, 12, distant from each other, a specific A/D converter and ECG signal conditioning device 14 which comprises a pacemaker detection circuit and which is connected to the electrodes and to a communication module 15 configured to communicate with the device 20 e.g. through a Bluetooth Low Energy link. The sensor comprises further a power controller 17 and a battery 18. The power controller can be connected to a charging base 19 possibly through an inductive charging circuit.

The electronic part of the sensor 10 may be embedded in a housing 13 from which protrude two flexible strips embedding electrodes wiring and having the electrodes 11, 12 at their ends. The length of the sensor from electrode to electrode is around 6 to 12 cm.

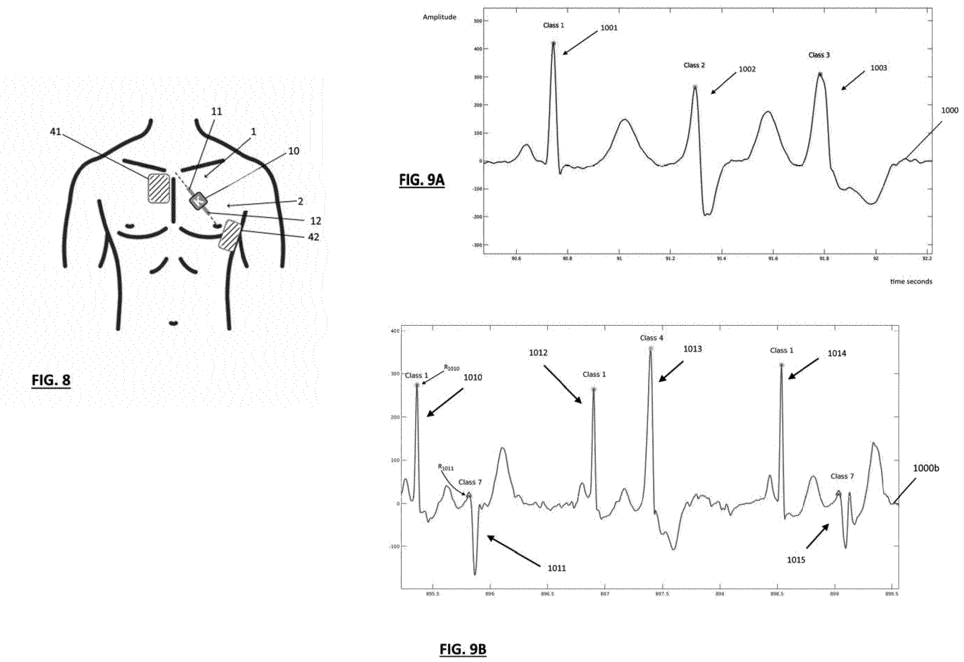

In the present disclosure a sensor with only two electrodes is used. As shown in figure 8, the electrodes 11, 12 of the sensor 10 are located in a MSN (Manubrium-Sternal-Nipple) orientation which is simple and informative. The sensor is located and centered on a straight-line segment directed from the Manubrium Sternal to the nipple. The sensor can be positioned on the patient's chest depending on the desired cardiac information. The sensor may be moved on such line towards the Manubrium of the Sternum, when extraction of the atrial 1 activity needs to be emphasized, or, towards the nipple when monitoring needs to be extended to the left ventricular apex 2.

Such positioning provides the following advantages:
- Easy anatomic location;
- Adapted to men and women;
- Pertinence of the delivered information as the sensor explores the atria, the septum, and the anterior wall of the heart;
- The sensor is outside of the areas 41, 42 intended to receive defibrillator pads which are needed in case of emergency.

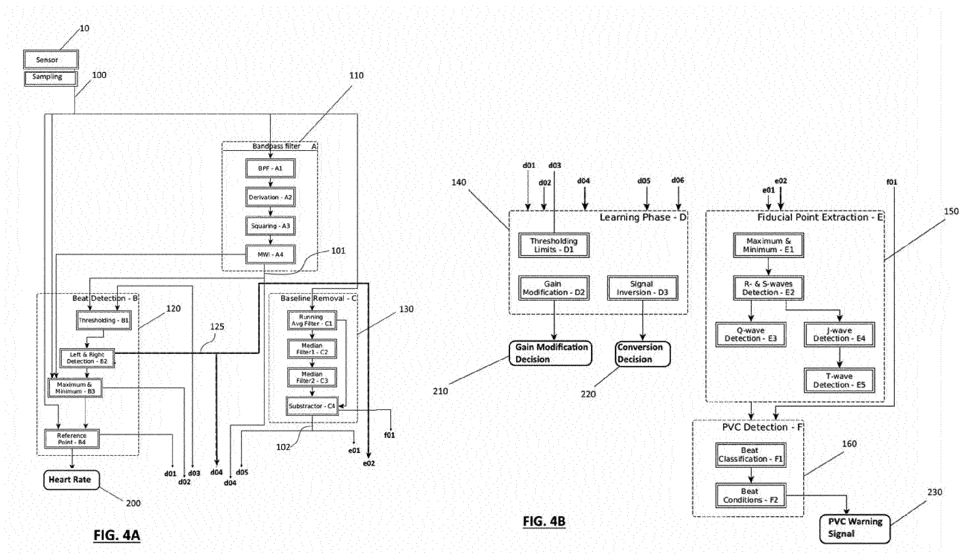

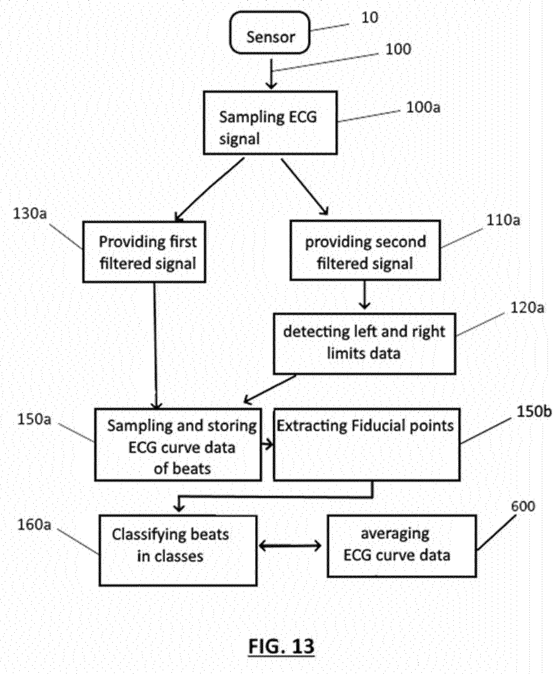

Figure 13 provides a general view of some features of the process for measuring heart beats fiducial points and classifying heart beats of the disclosure and figures 4A and 4B provides more details of such disclosure. In figure 13, the process comprises sampling in 100a a raw ECG signal 100 acquired with an ECG sensor 10 on a patient;
- providing in 130a a first filtered signal 102 through removing the baseline wander from said sampled raw ECG signal with a baseline removal filter module;
- providing in 110a a second filtered signal 101 through a bandpass filter module comprising a bandpass filter and derivation, squaring and moving window integration submodules providing a filtered signal for a beat detection module ;
- detecting in 120a left and right limits data for the beats in said second filtered signal within said beat detection module;
- in a Fiducial point extraction module, receiving the first filtered signal from the baseline filter removal module and receiving said left and right limits data from said beat detection module, sampling and storing in 150a ECG curve data of beats from said first filtered signal synchronized by said left and right limits data and extracting in 150b fiducial points of said beats from said ECG curve data 150, said fiducial points comprising at least the QRS points values of the beats, and classifying in 160a each of said beats in classes based on a correlation of its ECG curve data with respect to average ECG curves data of classes of previously averaged 600 classified beats.

Figures 4A and 4B provide more detailed description and additional features and describe various treatments of the signal starting from the raw ECG measurement sampled at a frequency of 200 to 300 Hz and preferably 250Hz for which up to 5mn of samples are stored first in first out. The signal processing of the raw ECG signal 100 obtained through the sensor 10 contains several modules described in figures 4A, 4B:
A first module represented in figure 4A is a specific digital bandpass filter 110 which is different from known 360Hz real time QRS detection algorithm used traditionally for beat detection.

The specific digital bandpass filter of the present disclosure is a stable finite impulse response filter of the 32^{nd} order having a frequency bandwidth of 8Hz to 25Hz using 33 coefficients. The filter delay is of only 64ms that is 16 samples for an ECG signal sampled at a frequency between 200 and 300 Hz and preferably 250Hz.

The filter is designed to allow a maximum attenuation of 5 dB in the passband, while it guarantees an attenuation of at least 55 dB in the stopband.

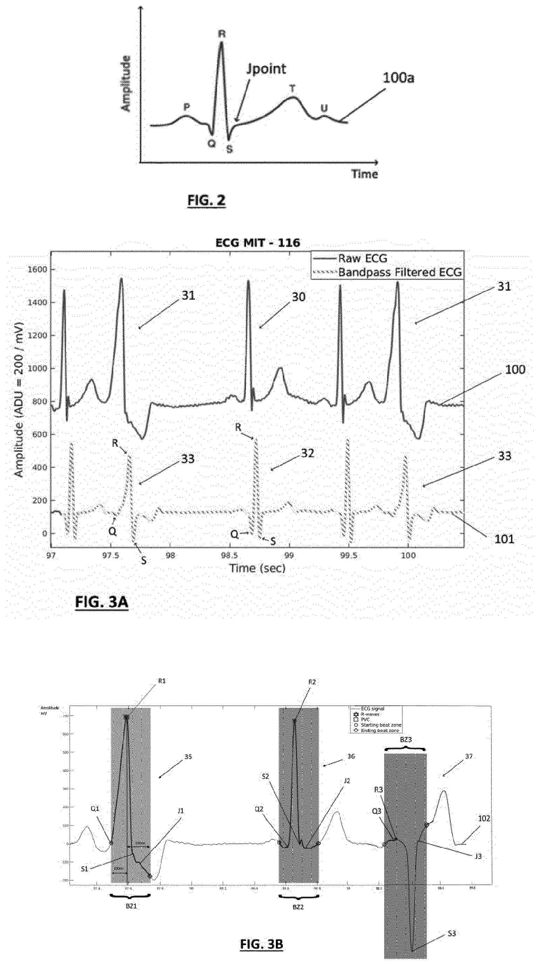

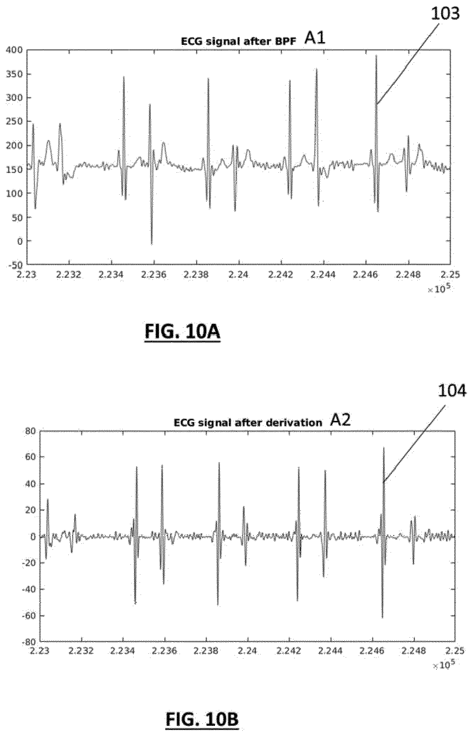

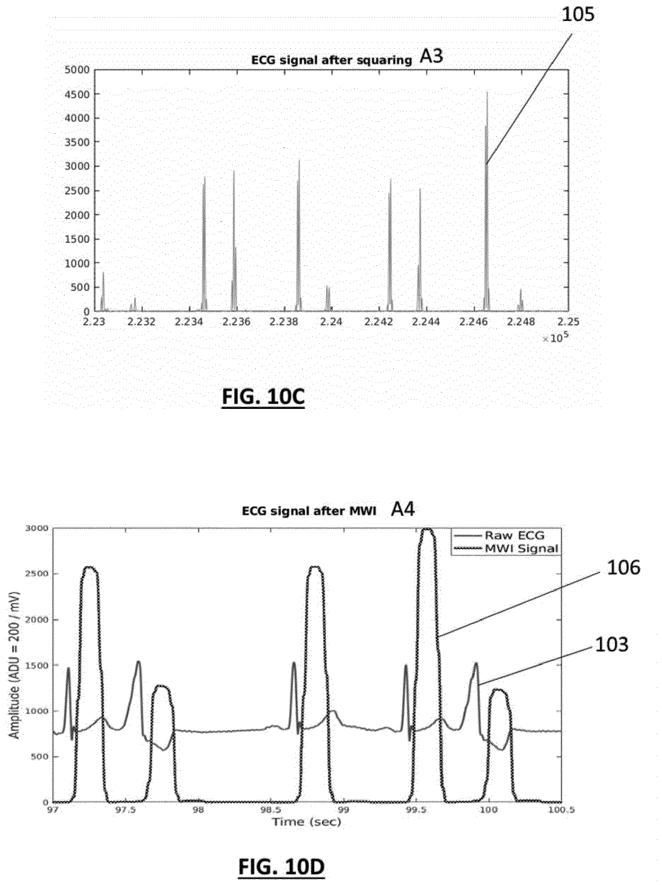

As shown on figure 3A, the main goal of the bandpass filter is to amplify the QRS complex curve when having both normal beats 30 and PVC beats 31 in order to improve detection of the R point and the starting and ending points of the beat curve. In figure 3A, the raw ECG signal 100 is shown above the resulting signal 101 upon applying the bandpass filter over such raw signal. In this figure, the difference between the signal 32 of a filtered standard beat 30 and the signal 33 of a filtered non-standard beat 31 can be seen. The signal used refers to the "physionet" database, and specifically to the MIT-BIH Arrhythmia database patient number 116. The bandpass filter amplifies the QRS complex curve of the signal. The filter module 110 comprises the bandpass filter module A1 by itself, a derivation A2 of the signal, a squaring A3 of the derived signal and moving window integration (MWI) A4. An example of signal curve 103 after bandpass filter module A1 is also given in figure 10A showing that the peak values are emphasized, the signal 104 after derivation A2 is shown in figure 10B, the signal 105 after squaring is shown in figure 10C and the signal 106 after the moving window integration A4 superposed to the signal after bandpass filter is shown in figure 10D. It should be noted that the signal 106 has a delay with respect to the signal 103 which has to be taken into account for the left/right detection discussed hereunder.

A second module represented in figure 4A is a beat detection module 120. As the QRS complex curve is enhanced by the bandpass filter for both normal and PVC beats, an accurate and fast beat detection is possible using traditional stages including thresholding B1, limits (left and right) detection B2 of the QRS complex curve, maximum value and minimum value measurement of the beat curve B3 which receives as input the raw ECG signal and the signal from the MWI of the Bandpass filter module 110, and a reference point setting B4. The reference point is considered as the absolute highest point in a QRS complex curve. This corresponds mainly to an R-wave summit when having a Rs form (big R small S), or and S-wave summit when having a rS for (small R big S). This module provides a heart rate value 200, left and right beat limits, and maximum/minimum values used in other modules.

The extraction of some fundamental features from biomedical signals is affected by noise, and hence accurate analysis may be difficult. Baseline wander in ECG signals is one such example, which can be caused by factors including respiration, variations in electrode impedance, and excessive body movements. Thus, removing the baseline wander is essential in order to have accurate features extraction.

In order to facilitate the fiducial point extraction in time-domain, the baseline wander caused by fluctuations such as breathing movements of the ECG signal is removed with a third module in figure 4A which is a baseline removal filter module 130 receiving the raw ECG signal as input and comprising a running average filter C1 followed by a first median filter C2 followed by a second median filter C3 and a subtractor C4 which subtracts the running average filter C1 data from the second median filter data.

An example process is explained hereunder.

The running average filter C1 is a filter of size 5 used at the beginning of this filtering procedure in order to remove high frequency components centered around 50 Hz.

The filter is a FIR and has the following coefficients:
b=[0.2 0.2 0.2 0.2 0.2].

In the second step in removing ECG baseline wander the filtered signal is passed the median filter C2 which is a 200ms median filte. This eliminates the QRS complex curve from the signal as the wider QRS complex curve doesn't exceed 200ms.

The upcoming step is to remove T-wave components. The first median filtered signal is again passed through a 600ms median filter C3 to eliminate the T-wave from the signal.

Then, the baseline wander can be eliminated via a simple subtraction of the output of the median filter C3 from the ECG signal.

A fourth Module represented in figure 4B is a learning phase module to adapt the gain of the ECG signal delivered by the sensor for this module. Such module provides a gain modification decision 210 and a conversion decision 220 to adapt the ECG signal. Such modules receive as inputs the data of the stages of the beat detection module 120, the output of the bandpass filter module 110 and of the subtractor stage C4 of the baseline removal module 130.

In the present application, a training period allows to determine a first dominant beat class. Such period of 10 seconds or 3 beats (the shortest is chosen) is used in order to determine a dominant beat class and to allow PVC (premature ventricular complex or contraction) detection starting from the 4th beat.

A fifth module represented in figure 4B is a Fiducial point extraction module 150 which comprises a time-domain analysis to extract the fiducial points Q, R, S, J and T-waves of a beat 34 of a regular ECG signal 100a such as disclosed in figure 2. This module receives as inputs the left and right detection limits data 125 from the beat detection module 120 and the subtractor output from the baseline removal module 130.

A first step E1 is detecting the maximum and minimum amplitude points of the curve to prepare R and S detection.

A second step E2 is the detection of the R- and S-waves of an ECG signal. Depending on the QRS complex curve form, the order of these two detections is different: if the form is Rs (big R-wave small S-wave) as beat curves 35 and 36 of figure 3B or beat curves 1010, 1012, 1013, 1014 of figure 9B, R-wave is detected first, and S-wave is detected second. If the form is rS (small R-wave, big S-wave) as beat curve 37 in figure 3B and curves 1011, 1015 of figure 9B, S-wave is detected first, and R-wave is detected second.

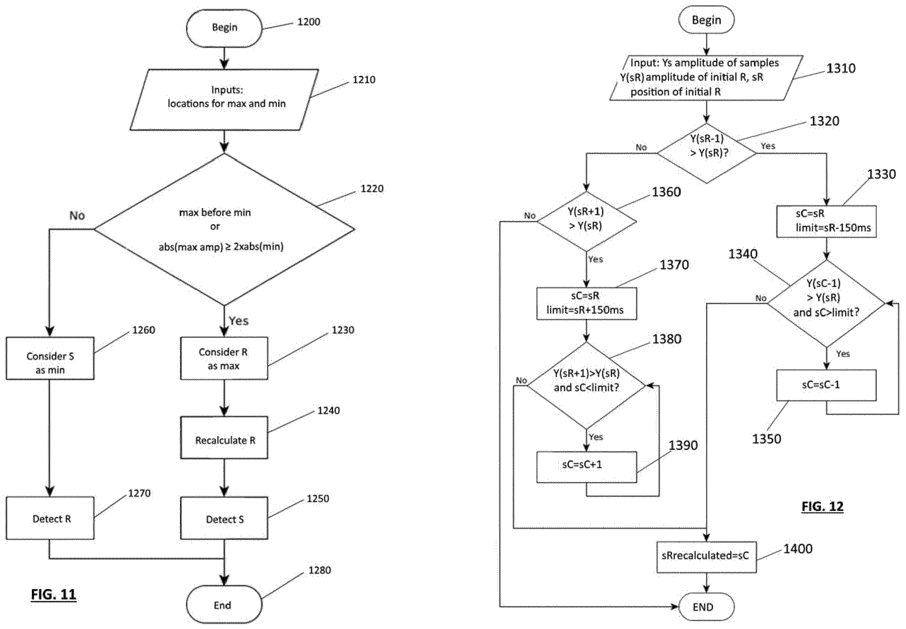

After detecting the maximum of the curve and minimum of the curve a RS detection 1200 shown in figure 11 is done.

This detection has in step 1210 inputs the locations of the max and min of the curve. In step 1220 one out of two conditions has to be accomplished in order to start the detection of R-wave prior S-wave in branch 1230, 1240, 1250:
a. Condition1: the global maximum between "left" and "right" lies before the global minimum;
b. condition 2: the absolute value of the global maximum is much greater (at least twice) than the absolute value of the global minimum.

Otherwise, in branch 1260, 1270 S-wave is detected first, and the R-wave is detected second.

### R-wave detection:

When a possible R maximum is detected first at step 1230, a verification step is still required, named "recalculate R" at step 1240. This verification is essential as the beat's limits "left" and "right" may both lie on the left side or the right side of the beat when having a PVC. Thus, the local maximum between such limits may be at the falling edge of the QRS (between R and S and not at the maximum R point of the R-wave), or at the rising edge of the QRS (between Q and R).

As described in figure 12, in order to improve detection of the maximum point of the R-wave, the amplitude of the sample before the initially detected R point is compared to the amplitude of the initially detected R point at step 1320. If this amplitude is higher than the initially detected R point, this check is repeated until the highest point of the curve is found with a limit of about 150ms or 37 samples before the initially detected R point at steps 1330, 1340, 1350 and the maximum amplitude point becomes the recalculated R point at step 1400.

If the amplitude of the sample before the initially detected R point is lower than the amplitude of the initially detected R point, the test is done with the sample after the initially detected R point at step 1360. If this amplitude is higher than the initially detected R point, this check is repeated until the highest point of the curve is found with a limit of about 150ms or more precisely 37 samples at a sample frequency of 250Hz after the initially detected R point at steps 1370, 1380, 1390 and the maximum amplitude point becomes the recalculated R point at step 1400.

On the other hand, when S is detected first, the R-wave maximum is considered as the highest point at the right side of R up to 120ms.

As stated earlier, the R-wave detection can be performed first when having a Rs form, or second when having a rS form.

### S-wave detection:

When a R-wave is detected first, S-wave detection 1250 starts from R-wave location up to an interval of 250ms. This allows considering large QRS when having premature ventricular contraction (PVC). The S-wave is considered as the first local minimum at the right side of R-wave. Two conditions are required prior considering an S-wave location: the first one is the change in slope for the falling edge of the QRS complex curve. This change must exceed 40% (value chosen after trial and error). Five samples are used as a distance between two points for the slope calculation.

When S is detected, some verification is required in order to avoid having false S-wave location especially when having a Right Bundle Branch Block (RBBB).

### RBBB first check:

The maximum (M) between the temporarily S and 40ms after its location is considered in order to verify whether a RBBB occurs or not. If the amplitude of S is positive, and the amplitude of M is greater than 50% of the amplitude of R, then a RBBB occurs and a new search for S starts. This new search has two parts that are performed consecutively:
Part1: a counter starting at the location of M, added to it 3 samples to avoid the problem of having flat signal at the peak. As long as the amplitude of the sample at the counter location is greater than zero, and the counter does not reach yet the 250ms limit, the counter is incremented by one.
Part2: as long as the counter does not reach the 250ms limit, and the next sample has lower amplitude than the current sample (at the counter), increment the counter by one.

### RBBB second check:

If the detected S-wave is closer than 40ms to the R-wave, a RBBB may occur. The maximum (M) between the S-wave location and S-wave plus 40ms to the right side of S point is detected. The minimum (m) between the M and 40ms to the right side of M is detected. If the RS amplitude is greater than 0.075mV (to avoid peaks at flat signal), and the amplitude of M-m is greater than 30% the amplitude of RS, then the new search for S starts at m to right side as long as the amplitude of the next sample is lower than the amplitude of the current sample. The 250ms limit has to be always respected. If the S-wave is detected at this stage, the code stops here.

### PVC check:

When a PVC occurs, large QRS and smooth S-wave will be present. Thus, verification is required in order to detect the S-wave when having a PVC.

The minimum m between the R-wave and 250ms after R is detected. If the amplitude of m is smaller than -0.2mV, and the amplitude of m is smaller than the amplitude of the previously detected S - 0.2mV, and if the amplitude of m is greater by more than 15% of the amplitude of the R-wave, then the S-wave location is considered to be m.

The amplitude of S-wave undergoes a final verification: if the amplitude of S is positive and greater than 20% the amplitude of the R-wave, starting with a counter at the S-wave location, two steps are required:
Step1: as long as the next sample is greater than zero, and as long as the 250ms limit is not reached, increment the counter by 1
Step2: as long as the amplitude of the next sample is smaller than the amplitude of the current sample, and the 250ms limit is not reached, increment the counter by 1.

After these two steps, the location of the S-wave is considered to be equal to the counter.

### J-wave detection E4:

Starting from S-wave location, the search interval for J-point is 150ms in order to include PVCs. Two conditions are considered in order to detect the J-point: the first one is that J-point has to be greater or equal to zero, and the second condition is when the slope from S-wave changes by at least 15%. Five samples are used for the slope calculation.

Another check is performed for the J-point detection in order to overcome large QRS when having a PVC. If the amplitude of the S-wave is smaller than -0.325mV and greater than 60% the amplitude of R-wave, and the amplitude of the J-point is smaller than -0.2mV, then J is considered as the next sample as long as this latter is negative, and the 150ms is not reached.

Once the J-point is detected, the T-wave search starts 40ms after J-point up to 600ms starting from the R-wave location, such that the search area does not exceed the "left" location of the next beat. Noteworthy to mention that the detection of the fiducial points for beat n starts when beat n+1 is received.

### T-wave detection E5:

Searching the T-wave starts 40ms after the J-point in order to avoid false T-wave detection when having negative J-point lower in absolute amplitude than negative T-wave.

The T-wave is considered as the global maximum or minimum that stays maximum or minimum respectively for 50ms in both sides. The minimum is taken into account in order to detect the negative T-waves.

If the search limit (stop) is before the search start which depends on the next beat, the search will stop 400ms after the start. The 400ms are considered as ST segment duration (up to 150ms) + the T-wave duration (up to 250ms).

If the searching interval is too short (50ms), T is considered as the most far point.

If more than one peak fulfills the condition of being maximum for at least 50ms from both sides, left and right, then the peak with the highest amplitude in absolute value is considered to be T.

### Q-wave detection E3:

The search for the Q-wave starts from the position of the R-wave up to 150ms to the left, such that this duration does not exceed the "right" limit of the previous beat. Normal QRS duration varies between 80 and 120ms, however, the value 150ms is chosen after trial and error in order to include PVCs and Left Bundle Branch Block (LBBB).

If the R-wave position is too close (25ms) to the right of the previous beat, the right is considered as the Q-wave location for the current beat and the search is stopped.

The Q-wave is considered as the first local minimum at the left side of the R-wave. Two conditions are required prior considering a Q-wave location: the first one is the change in slope for the falling edge of the QRS complex curve. According to the measurements done, this change must exceed 10%. Five samples are used for the slope calculation. The location of Q-wave is decreased by two samples in order to be closer to the Q-on position. Note that many ECG signal do not contain both Q-on and Q-off points.

If the Q-wave location is found to be too close from the R-wave (up to 50ms), an LBBB may occur. The maximum between the Q-wave and the 50ms that precedes R-wave is found. If the value of this local maxima is greater by at least 30% (after trial and error) of the RS amplitude, and if the Q-wave is found to be positive, then a LBBB occurs. The search for the true Q-wave starts again starting from this local maximum.

The second condition to be verified is if the Q-wave amplitude is not positive and lower by at least 30% (after trial and error) than the RS amplitude. This condition is considered when having a LBBB form.

Another check is verified on the Q-wave detection when it is found to be greater than zero, and its amplitude exceeds 20% of both the amplitude of the R-wave and the absolute amplitude of the S-wave. If this condition is true, Q-wave is considered as the first point at the right side of R having a non-positive value.

The Q-wave detection technique takes into consideration this special case in order to avoid false Q-wave detection.

The Q-wave can be detected in three different cases: the first one is when having sharp Q-wave with negative value, the second case is when having a RBBB with smooth positive Q-wave, and the third case when having a positive smooth positive Q-wave.

A sixth module represented in figure 4B is the PVC detection module 160 which takes into consideration the time-domain analysis of the ECG signals including the fiducial points locations and measurements such as QRS duration and T-wave and RS amplitudes to define beats classification and beats conditions as it will be discussed hereunder. Such module receives as inputs the fiducial point extraction module 150 output, that is curve data comprising the fiducial points time location and amplitude, and the baseline removal module 130 output.

The present application uses time-domain analysis of the signal and tested over all MIT-BIH Arrythmia database ECG signal, see Moody GB, Mark RG. "The impact of the MIT-BIH Arrhythmia Database". IEEE Eng in Med and Biol 20(3):45-50 (May-June 2001). (PMID: 11446209)

An important feature of the present application is PVC detection in module 160.

The detection of the PVC signals contains two main parts, beat classification and PVC conditions.

### Beat Classification

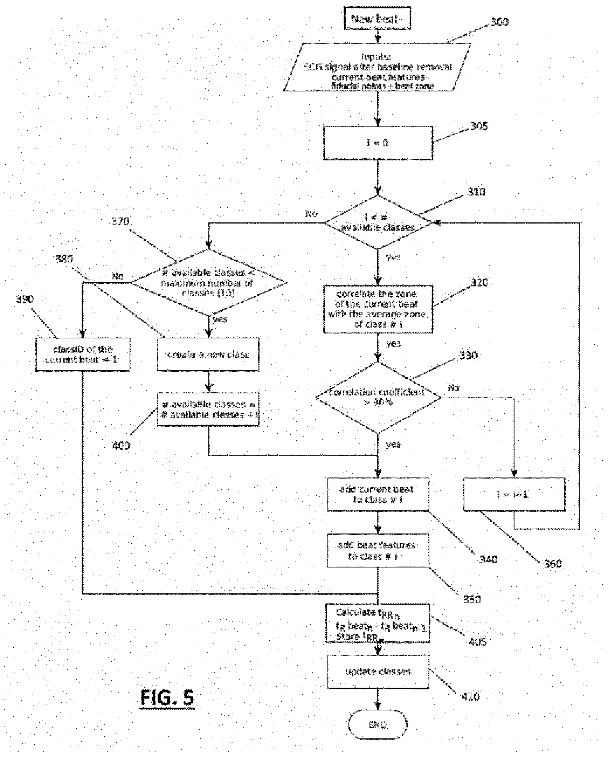

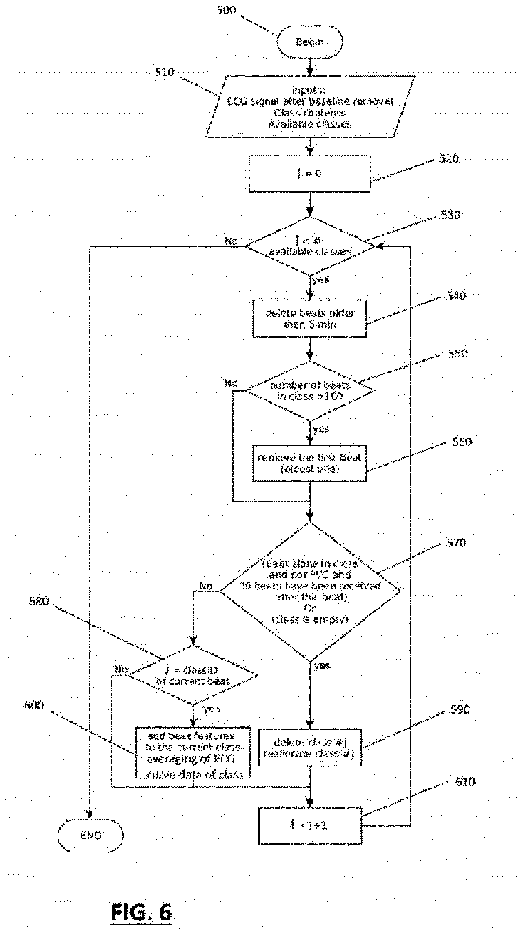

The beat classification aims to classify every beat based on its shape in order to determine the dominant QRS curve form. This helps identifying several beat signals abnormalities including PVC, bigeminy, trigeminy, multiform PVC, etc. In this regard, beat classification allows providing a detection of multiform PVC when several classes of beats correspond to differently shaped PVC beats within the stipulated 5mn rolling time window. The flowchart presented in figure 5 shows the process of classifying a beat, while the flowchart in figure 6 shows how a class is updated after receiving a new beat.

The classification is based on correlating a beat with all available classes in order to determine the most similar class for this beat. Correlation is done on a Beat Zone as disclosed in figure 3B where two different beats are shown on a signal 102 with baseline wander removed. The Beat Zone is issued after detecting R-wave and it uses the R-wave location and the ECG signal after removing baseline variation.

### Beat Zone selection:

When a beat is detected, its features are extracted. The goal of this step is to set the data zone or Beat Zone for each beat in order to classify the beat in later step. The QRS complex duration for a normal beat ranges between 80ms and 120ms. Thus, and in order to select the appropriate zone for storing data of a beat, a window of 250ms around R-wave is chosen. The window is determined after the detection of the R-wave of beats as R1, R2, R3 respectively for beats 35, 36 and 37 in figure 3B where Beat Zones BZ1, BZ2, and BZ3 are defined on beats 35, 36 with positive high level R-wave and small S-wave and 37 which has a nearly null R-wave and negative high level S-wave. This zone duration is divided in two parts, preceding and following the R-wave. The sub-zone preceding R-wave is chosen to have 100ms length, while the sub-zone following the R-wave is chosen to be 150ms. This guarantees covering the normal and abnormal QRS complexes, and thus including Q, R, S points of the ECG complex. Figure 3B shows approximated Q1, R1 and S1 for beat 35, approximated Q2, R2 and S2 for beat 36, approximated Q3, R3 and S3 for beat 37. Such figure also represents the J-point after the end of the QRS complex curve, J1 for beat 35, J2 for beat 36 and J3 for beat 37. In addition, the selected zone area with a maximum length between two R-waves in the beat zone of 150ms avoids having overlap beats even when the heart rate reaches 300bpm which results in a distance of 200ms between two consecutive R-waves. This is done on stored samples of the curve which are analyzed and treated by a signal treatment software in the device of the invention. In addition, the stored data of the sampled beat curve is used to compute the correlation of such data with beat classes in order to select the correct beat class for the beat.

To obtain data representative of the evolution of the patient's cardiac condition, storage the last 5 minutes of the beats data within is done first in first out.

### Class selection:

As shown in figure 5, when a beat is received, its fiducial values such as QRS complex curve duration, QRSJ duration, absolute T-wave amplitude, RR interval, RS amplitude, R-wave amplitude, S-wave amplitude, and Beat Zone, referred to as beat features hereunder, are calculated at step 300. A process to classify the beat comprises setting a class counter i=0 at step 305 and entering a loop which comprises checking that the class number is smaller than the available classes number # at step 310, comparing the Beat Zone values through a correlation calculation at step 320 and checking the correlation coefficient at step 330. If the correlation coefficient is greater than 90% the beat and its features are added to the class number i at steps 340, 350. If the correlation coefficient is less or equal to 90% the beat does not belong to the currently checked class i and i is incremented by 1 at step 360 to prepare a comparison with the next class except if the number of classes available is reached at 310. In such case a test is made at 370 to check if the maximum number of classes is reached in order either to create a new class at 380, increase the classes number # by one at step 400 and add the current beat and beat features to the new class at 340, 350 or, in case the number of classes has reached its maximum which in the present example is set at 10 classes, the beat is identified as class ID -1 at 390 for specific handling. After the beat is classified the classes are updated at 410 and the process is ended until next beat.

To summarize, in order to add the zone area of the new beat to the correct class, a matrix is used in order to save the zones of all beats of a class. The average of this matrix (beats zones) is correlated with the new beat for class selection.

Note that in the given example classes are numbered from 0 to 9 as the maximum number of classes is set to 10 classes.

Usual ECG signals with multiform PVCs may contain up to four different forms: one for a normal beat and three for different PVCs. However, a noisy signal may result in having many cases due to false positive beats. In order to avoid having infinite number of classes due to noisy signals, a class elimination process is used. This process allows the deletion of a class whenever only one non-PVC beat belongs to it, and 10 beats have been received since this beat without updating its class.

Note that the system is also configured preferably to remove beats having an amplitude lower than 0.15 mv as specified by the standard 60601-2-27.

### Updating classes:

When a beat is classified, its features are added to the corresponding class. Each class contains several parameters that will be useful in distinguishing between normal and abnormal beats. These parameters include QRS complex curve duration, QRSJ curve duration, absolute T-wave amplitude, RR interval, RS amplitude, R-wave amplitude, and S-wave amplitude. After the class selection decision of figure 5, the class to which the new beat belongs and other is updated. This includes the following steps disclosed in figure 6:

In step 510 the data needed as input are the ECG signal after baseline removal of module 130 of figure 4A, the classes contents, and the available classes.

In step 520 a counter j is set to 0 and a loop to update the classes is initiated.

In such loop ending at 530 when j reaches the number of available classes, a first step 540 is deleting beats older than 5min, then at steps 550, 560 the oldest beat is removed if the number of beats is above 100. This allows updating the dominant class (and other classes as well) whenever the QRS curve shape changes smoothly over time. This is useful especially when having changes in the ST-segment.

Then a test is done at 570 to check for empty classes or classes where a beat which is not PVC is alone while 10 beats have been received after such beat. Classes satisfying such conditions are deleted at and their ID is reallocated at steps 570, 590.

For other classes, a test 580 is done to check if the class ID j is the current beat class and if yes the current beat features are added to the class at 600. When all classes have been treated, the process is ended.

Examples of results of beats' classification are given in figures 9A, 9B for several types of beat shapes where the variation of the baseline has been removed.

In figure 9A, three beats 1001, 1002, 1003 on a curve 1000a are classified in three classes. The first beat 1001 is a normal beat while the second beat 1002 and third beat 1003 have abnormal shapes.

In figure 9B, six beats are classified on a curve 1000b, beat 1010,1012 and 1014 belong to a first class which is the dominant class, beats 1011 and 1015 belong to a class number 7 and beat 1013 belong to a class number 4. In such case, other classes exist from previously detected beat shapes.

This permits the detection of multiform PVC when several classes of abnormal beats are found in the last 5 minutes.

It should be noted that the dominant class is statistically a class of normal beats as the number of abnormal beats has a physiological limit above which tragic consequences such as fibrillation or heart attack occur.

In the present application, the dominant beat class becomes a reference class. ≥

### PVC conditions:

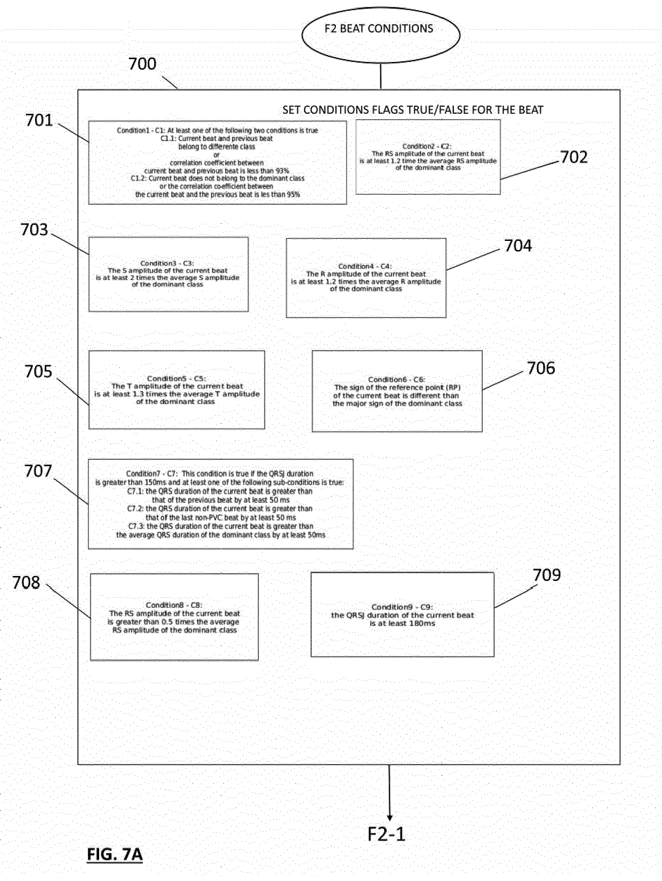

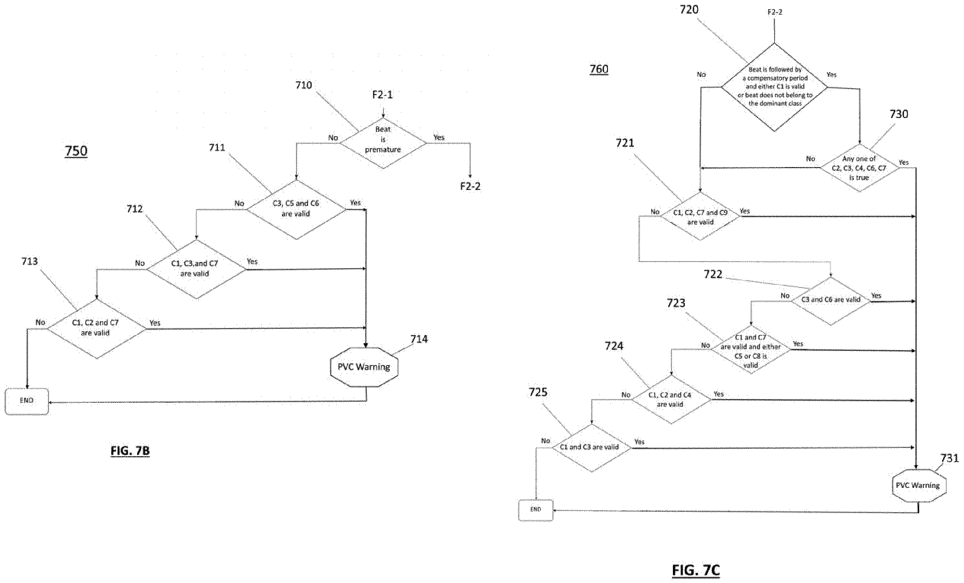

Figures 7A, 7B and 7C disclose steps to provide an abnormal beat warning taking into consideration the shape changes in an abnormal beat in both time-axis and amplitude-axis. These detection criteria are tested over all MIT-BIH Arrythmia database.

In figure 7A and in a first step 700 comprising several subparts to provide flags based on several conditions, the beat curves are analyzed in order to check the validity of a series of conditions and provide a false/true matrix of such conditions for the beats.

As said before, all beats within the last 5 minutes are saved. In addition, for each beat a flag telling whether it is an abnormal PVC beat or not is stored together with the beat values.

The conditions are as follows:
Condition C1 701 is related to the class of the beat under test.
Condition C1 is true if at least one of the following two conditions is true:
   Subcondition C1.1: The current beat and the previous beat belong to different classes OR the correlation coefficient between the two is less than 93%;
   Subcondition C1.2: the current beat is not part of the dominant class OR the correlation between the current beat and the previous beat is less than 95%.
Condition C2 702 concerns the amplitude of the RS part of the beat curve (RS): Condition C2 is true if the RS amplitude of the current beat is at least 1.2 times greater than the average RS amplitude of the dominant class of beats;
Condition C3 703 is true if the amplitude S of the current beat is at least 2 times smaller than the average amplitude of the S-waves of the dominant class;
Condition C4 704 is true if the amplitude R of the current beat is at least 1.2 times greater than the average R amplitude of the dominant class;
Condition C5 705 is true if the amplitude T of the current beat is at least 1.3 times greater than the average amplitude of the T waves of the dominant class;
Condition C6 706 is true if the sign of the reference point of the current beat (RP) is different from the major sign of the dominant class;
Condition C7 707 is true if the duration of the QRSJ is greater than 150ms and at least one of the following 3 subconditions is valid:
   - Subcondition C7.1: The duration of the QRS curve of the current beat is greater than that of the previous beat by at least 50ms;
   - Subcondition C7.2: the duration of the QRS curve of the current beat is greater than that of the last non-PVC beat by at least 50ms;
   - Subcondition C7.3: the duration of the QRS curve of the current beat is greater than that of the average QRS curve duration of the dominant class by at least 50ms.
Condition C8 708 is true if the RS amplitude of the current beat is greater than 50% of the average RS amplitude of the dominant class;
Condition C9 709 is true if the QRSJ curve duration of the current beat is at least 180ms.

After setting the condition flags, the PVC warning flag is set according to the following process. This process determines whether a beat is an abnormal beat indicating potential PVC or not by taking into account the conditions of the beat, its class, as well as its prematurity and compensatory rest.

Concerning the premature condition, a beat is considered as premature if the time between the R point of a previous beat and the R point of the current beat is greater than the mean R to R time of the three previous beats. This is done with calculating t_{RR} between current beat n calculated R point and previous beat n-1 calculated R point and comparing the current point t_{RRn} to the average t_{RRm} of the three previously calculated and stored time between R points (t_{RRn-i}, t_{RRn-2}, t_{RRn-3})/3 for providing a premature beat flag. The premature beat flag is provided when a last t_{RR} is smaller than t_{RRM}x0.875.

In figure 7B, in case of a beat which is not premature, the PVC warning flag 714 is on when:
In test 711 C3, C5, and C6 are valid;
In test 712, C1, C3, and C7 are valid;
In test 713, C1, C3, and C7 are valid.

Then in figure 7C in case of a beat which is premature, the PVC warning flag 731 is on if one of the following criteria is valid:
If at step 720 the premature beat is followed with a compensatory rest and either condition C1 is valid or the beat is in the dominant class then if at 730 any one of condition C2, C3, C4, C6 or C7 is true;
If at step 720 the premature beat is followed with a compensatory rest and either condition C1 is valid or the beat does not belong to the dominant class, the flag is on if any one of the conditions C2, C3, C4, C6, C7.

If at step 720 the condition is not true, the flag is on in case
C1, C2, C7, and C9 are valid at step 721;
C3 and C6 are valid at step 722;
C1 and C7 are valid and either C5 or C8 is valid at step 723;
C1, C2, and C4 are valid at step 724;
C1 and C3 are valid at step 725.

The warning flags are displayed on the screen 22 of the computer device 20 and a warning sound may be emitted by the speaker 24 of such device.

In the present invention which is not limited by the here above description since other tests may be implemented on beat shapes and class monitoring, the process is preferably implemented on a standalone device comprising the bipolar sensor 10 and a tablet PC 20 which are easily transported for use in emergency vehicles, on battlefields or in case of emergency conditions such as accidents or catastrophes. As discussed above, an advantage of the process and device disclosed is that it permits to use a simple and easy to use bipolar sensor 10 which can be located in a Manubrium Sternal - nipple position or other positions on the torso of a patient as disclosed in figure 8 leaving space for defibrillator pads 41. As the communication between the sensor and tablet is a radio communication such as Bluetooth, cables are not needed allowing the medical staff to move easily around the patient and proceed to the needed treatments on the patient.

## Claims

1. Process for measuring heart beats fiducial points and classifying heart beats, **characterized in that** it comprises sampling (100a) a raw ECG signal (100) acquired with an ECG sensor (10) on a patient,
- providing (130a) a first filtered signal (102) through removing the baseline wander from said sampled raw ECG signal with a baseline removal filter module (130),
- providing (110a) a second filtered signal (101) through a bandpass filter module (110) comprising a bandpass filter and derivation, squaring and moving window integration submodules providing a filtered signal for a beat detection module (120)
- detecting (120a) left and right limits data (125) for the beats in said second filtered signal within said beat detection module,
- in a Fiducial point extraction module (150), receiving the first filtered signal (102) from the baseline filter removal module (130) and receiving said left and right limits data (125) from said beat detection module (120), sampling and storing (150a) ECG curve data of beats from said first filtered signal synchronized by said left and right limits data and extracting (150b) fiducial points of said beats from said ECG curve data (150), said fiducial points comprising at least the QRS points values of the beats, and classifying (160a) each of said beats (1001, 1002, 1003, 1010, 1011, 1012, 1013, 1014, 1015) in classes based on a correlation of its ECG curve data with respect to average ECG curves data of classes of previously averaged (600) classified beats.

2. Process for measuring heart beats fiducial points and classifying heart beats according to claim 1 wherein the sensor (10) being a two electrodes ECG, said sensor is positioned on the chest of the patient in a Manubrium-Sternal-Nipple orientation.

3. Process for measuring heart beats fiducial points and classifying heart beats according to claim 1 or 2 wherein said fiducial points extraction comprise further at least one of the maximum and minimum amplitude values of a beat, J-point through J-wave and T-wave extraction.

4. Process for measuring heart beats fiducial points and classifying heart beats according to claim 1, 2 or 3 wherein sampling and storing ECG curve data of beats comprises detecting a R point of said beats, setting a window (BZ1, BZ2, BZ3) around the detected R (R1, R2, R3, R₁₀₁₀, R₁₀₁₁) point of said beats, said window providing a Beat Zone having two sub-zones for covering the QRS complex curve of said beats, the process comprising analysing the sampled and stored ECG curve data during said Beat Zone to extract said fiducial points within said Beat Zone.

5. Process for measuring heart beats fiducial points and classifying heart beats according to claim 4 wherein said Beat Zone (BZ1, BZ2, BZ3) is defined to have a width sufficient to include a QRS complex of 80ms to 120ms including the J-point of an ECG complex after the end of the QRS curve at usual heart rates such width being further defined to avoid to take into account two beats with a beat period at a theoretical maximum of 300 beats per minute.

6. Process for measuring heart beats fiducial points and classifying heart beats according to claim 5 wherein said Beat Zone is a time window having a length between 200ms and 400ms and preferably 250ms, having a first sub-zone of 100ms length preceding a calculated R-wave maximum point, and having a second sub-zone of 150ms following said calculated R-wave maximum point.

7. Process for measuring heart beats fiducial points and classifying heart beats according to any one of claims 1 to 6 wherein classifying said beats comprises a first learning phase (140) comprising storing sampled ECG curve data of Beat Zones and calculating fiducial points of a limited number of beats from such ECG curve data to provide initial beat classes.

8. Process for measuring heart beats fiducial points and classifying heart beats according to claim 7 comprising providing a first dominant beat class in said learning phase.

9. Process for measuring heart beats fiducial points and classifying heart beats according to claim 7 or 8 wherein classifying said beats comprises for subsequent beats after the learning phase, correlating (320) the ECG curve data of the Beat Zone of a new beat with the average values of ECG curve data of the Beat Zone of beats in previous classes to determine the most similar class for this beat and wherein:
- in case of correlation of the Beat Zone of such new beat with existing classes is lower than a limit value (320), and the maximum number of classes is not exceeded, a new class is created (380) and the new beat becomes a member of it as long as the maximum number of classes is not reached;
- in case of correlation of the Beat Zone of such new beat with existing classes is lower than a limit value and the maximum number of classes is reached and the new beat does not match any of the existing classes, the new beat remains without class and receives a flag (390);
- in case of correlation of the Beat Zone of such new beat with an existing class is higher than a limit value, the new beat is added to the class (340) and the average values of ECG curve data of the class is recalculated.

10. Process for measuring heart beats fiducial points and classifying heart beats according to any one of the preceding claims wherein said bandpass filter (110) is a stable finite impulse response filter of the 32nd order type.

11. Process for measuring heart beats fiducial points and classifying heart beats according to claim 10 where said bandpass filter is designed to order to have a frequency bandwidth of 8Hz to 25Hz using 33 coefficients and a filter delay is of less than 16 samples for an ECG signal sampled at 250Hz.

12. Process for measuring heart beats fiducial points and classifying heart beats according to any one of the preceding claims comprising determining a dominant class, said dominant class comprising the higher number of classified beats in a rolling time window.

13. Process for measuring heart beats fiducial points and classifying heart beats according to claim 12 wherein:
- said rolling time window is 5min (540);
- and/or the classes are limited to 100 beats, first in first out (560);
- and/or an empty class or a class having only one beat after receipt of ten new beats is removed from the classification (570, 590).

14. Process for detecting abnormal beats comprising the process of measuring heart beats fiducial points and classifying heart beats according to claim 12 or 13 and comprising further comparing (700) ECG curve data of the Beat Zone of a current beat with a set of conditions with respect to average Beat Zone ECG curve data of the dominant class and/or previous beats to provide a set of flags for abnormal beats.

15. Process for detecting abnormal beats according to claim 14 comprising further calculating and storing the time t_{RR} between current beat calculated R point and previous beat calculated R point (405) and comparing (710) the current point t_{RR} to the average t_{RRm} of the three previously calculated and stored time between R points (t_{RRn-1}, t_{RRn-2}, t_{RRn-3})/3 for providing a premature beat flag.

16. Process for detecting abnormal beats according to claim 15 wherein said premature beat flag is provided when a last t_{RR} is smaller than t_{RRM}x0.875.

17. Process for detecting abnormal beats according to claim 14 comprising a validation tree (750, 760) combining said premature beat flag and flags of said set of flags for abnormal beats to raise warning flags (714, 731) upon premature ventricular contraction detections.

18. Process for detecting abnormal beats according to any one of claims 14 to 17 wherein a multiform PVC warning flag is set upon detection of more than one PVC class of beats is detected within said rolling time window.

19. Device for implementing the process of claims 1 to 18 comprising an ECG sensor (10) and a computer device (20), said ECG sensor being provided with two electrodes (11, 12) adapted to measure electrical heartbeat signals on the skin of a patient and connected to said computer device (20), said computer device being configured to communicate with said sensor and to compute said numerical representation according to said process.

20. Device according to claim 19 wherein said computer device (20) comprises further display means (22) provided for displaying said numerical representation of beat curves, classes of beats and warning messages upon detection of occurrences of abnormal beat curves.

21. Device according to claim 19 or 20 wherein said ECG sensor is a remote ECG sensor (10) provided with two electrodes (11, 12) adapted to measure electrical heartbeat signals on the skin of a patient and provided with an analog to digital converter (14) and radio communication circuits (15) to connect through radio communication said sensor to said computer device (20) and transmit numerical representations of said heartbeat signals to said computer device (20).

22. Computer program comprising instructions for implementing the process of any one of claims 1 to 18 when such program is executed by a computer.

23. Non transitory medium readable by a computer on which is recorded a program for implemented the process of any one of claims 1 to 18 when such program is executed by a computer.
